## Europäisches Patentamt
## European Patent Office
## Office européen des brevets

(19)

(11) Numéro de publication: **0 097 095**
A1

# DEMANDE DE BREVET EUROPEEN

(12)

(21) Numéro de dépôt: **83401165.2**

(22) Date de dépôt: **08.06.83**

(51) Int. Cl.³: **A 61 L 9/04, A 61 L 9/01**

(30) Priorité: **09.06.82 FR 8210042**

(71) Demandeur: **Goutal épouse Meunier, Annick, 17, rue des Boulangers, F-75005 Paris (FR)**
Demandeur: **Proux épouse Perrot, Micheline, 80, rue de l'Université, F-75007 Paris (FR)**

(43) Date de publication de la demande: **28.12.83 Bulletin 83/52**

(72) Inventeur: **Goutal épouse Meunier, Annick, 17, rue des Boulangers, F-75005 Paris (FR)**
Inventeur: **Proux épouse Perrot, Micheline, 80, rue de l'Université, F-75007 Paris (FR)**

(84) Etats contractants désignés: **BE CH DE FR GB IT LI**

(74) Mandataire: **Bloch, Robert et al, 39 avenue de Friedland, F-75008 Paris (FR)**

(54) **Nouveau corps odorant.**

(57) Corps odorant caractérisé par le faut qu'il est constitué de pierres poreuses de granulométrie variable suivant les applications imbibées d'un mélange d'un parfum et d'un alcool saturé inférieur, en proportion de 40 à 60 % en poids, rapportée au melange.

EP 0 097 095 A1

Nouveau corps odorant.

Depuis des siècles, il a été d'usage de parfumer les sanctuaires, les lieux du culte, puis les pièces de réception et enfin les pièces d'habitation. On utilisait généralement l'encens ou la myrrhe ou d'autres graines ou arbustes que l'on faisait brûler lentement pour dégager les essences odorantes.

Plus tard, on a utilisé l'odeur de certaines cires pour la fabrication de bougies odorantes et ce procédé a ensuite été repris par les parfumeurs qui ont intégré les parfums dans des cires non odorantes à l'origine.

Il a enfin été très traditionnel de parfumer les placards à linge en y laissant séjourner des sachets en tissus ou des boules perforées en porcelaine remplis de graines de lavande ou de pétales de fleurs.

Enfin, on a parfumé, ou plutôt désodorisé, les ambiances par gaz parfumés avec aérosols.

Ces procédés anciens présentaient certains inconvénients :

- ceux basés sur la combustion dégagent une odeur secondaire propre à la combustion des matières organiques et leur durée est assez limitée, nécessitant une recharge fréquente;
- les sachets de lavande ou de pétales de fleurs ont un dégagement d'odeur très faible et d'une très courte durée;
- l'utilisation des aérosols conduit à l'utilisation de produits chimiques synthétiques nocifs.

La présente invention vise à pallier les inconvénients rappelés ci-dessus et à procurer un corps très odorant, dégageant un parfum d'ambiance, sans combustion propre et demeurant odorant avec intensité pendant longtemps, en principe plusieur mois.

Ce but est atteint, selon l'invention, avec un corps constitué de poudre ou de pierres poreuses, en particulier de diatomites (ou terre d'infusoires ou kieselguhr) imbibées d'un mélange d'un parfum et d'un alcool saturé inférieur, de préférence ayant au plus 4 atomes de carbone, en particulier de monopropylèneglycol, en proportion de 40 à 60% en poids, rapportée au mélange.

L'adjonction au parfum d'alcool saturé inférieur, notamment de monopropylèneglycol, a pour effet de limiter la vitesse d'évaporation de l'essence parfumée, ce qui permet au mélange laissé à l'air libre de rester odorant pendant plusieurs mois, alors que, sans cette adjonction, le parfum serait éventé au bout de quelques semaines.

Les pierres poreuses, ou la poudre obtenue à partir de ces mêmes pierres qui sont imbibées du mélange parfumé, jouent, grâce à leur perméabilité et leur faculté d'absorption des liquides, le rôle de réservoirs à capacité microscopiques multiples.

La pierre poreuse préférée, la diatomite, est une roche blanche légère, très poreuse et constitue une roche réservoir idéale pour cette application. Elle est constituée de silice hydratée provenant de la fossilisation d'algues unicellulaires dont la membrane cellulosique a fixé la silice de l'eau dans laquelle elles étaient immergées. Ces fossiles se sont accumulés dans certains fonds de lacs ou de mers et leurs gisements se sont rapprochés de la surface du sol à la suite de mouvements géologiques.

On en trouve dans de nombreuses régions en France (Massif Central), en Italie (Carrare), en Allemagne (Duché de Bade), en Autriche (Bas Tyrol) et aux U.S.A. (Nevada).

Ces roches, après traitement les ayant débarassé de leurs impuretés et humidité par recuit à haute température, constituent une matière chimiquement inerte, d'une grande perméabilité, d'où une grande faculté d'absorption des liquides.

D'autres roches poreuses, naturelles ou synthétiques (pierre ponce, alumine, billes de verres expanse...) peuvent donner des résultats analogues, mais nettement moins performants sur le plan de l'absorption, leur constitution n'ayant pas la finesse de celle de la diatomite.

Suivant le volume dont on désire parfumer l'atmosphère, on pourra utiliser les pierres, ou cailloux, poreuses imprégnées sous deux formes :

a) pour des volumes relativement petits tels que placards, pièces de rangement, salles de bains, petits dégagements, véhicules automobiles, on utilisera avantageusement la poudre ou les cailloux dans des petits récipients dont on ouvre le couvercle lorsque l'on désire le dégagement du parfum, ou bien les cailloux sont soit à l'air libre, soit dans des sachets de tissus à mailles suffisamment larges pour laisser librement passer les effluves contenant de 50 à 100 g de pierres. Le tissu de ces sachets pourra présenter un aspect esthétique attractif de par dessin et coloris.

b) pour des volumes plus importants, tels que salon, salle à manger, chambre à coucher, on pourra avantageusement chauffer ces cailloux pour en accélérer et augmenter le dégagement d'odeur. On pourra, dans ce but, placer quelques cailloux de 5 à 20 g environ suivant la taille de la pièce sur un support chauffant pouvant être, soit :

- une armature fixée sur une lampe électrique, soit :
- un élément en terre cuite ou en verre réfractaire posé sur une surface chauffante (lampe, radiateur, poële...)

soit encore :

- un élément constitué par une prise électrique alimentant une résistance noyée dans une porcelaine réfractaire, ou :

- un ensemble constitué par un élément chauffant et un petit ventilateur électrique.

Le chauffage de ces cailloux accélérant la vitesse d'émanation, donc diminuant la durée d'activité de ces pierres odorantes, on pourra réactiver ces pierres en les imbibant à nouveau du mélange parfum-alcool dont des doses prêtes à l'emploi auront été fournies avec les pierres elles-mêmes.

Ces exemples d'utilisation et de supports sont donnés à titre d'exemple et ne sont pas limitatifs.

On donne ci-après un exemple de préparation d'un corps odorant selon l'invention.

On met en contact dans un récipient, en acier inoxydable ou en aluminium, dans la proportion de 1 kg de pierres de diatomite et de 0,6 à 1 kg de mélange parfum, on agite de temps en temps pour obtenir une répartition égale du parfum dans les pierres et on laisse séjourner de 3 à 5 jours pour obtenir une absorption totale du parfum, jusqu'à ce que les cailloux ne présentent plus aucune trace d'humidité.

Le mélange parfum est constitué du parfum de base lui-même et de monopropylèneglycol dans la proportion de 30% à 60%, mélange effectué à froid.

Les proportions cailloux-mélange parfum et les proportions parfum-alcool sont fonction du parfum de base que l'on utilise et de la rétention que l'on désire obtenir.

Revendications

1.- Corps odorant caractérisé par le fait qu'il est constitué de pierres poreuses de granulométrie variable suivant les applications imbibées d'un mélange d'un parfum et d'un alcool saturé inférieur en proportion de 40 à 60% en poids, rapportée au mélange.

2.- Corps odorant selon la revendication 1, dans lequel les pierres poreuses sont des diatomites.

3.- Corps odorant selon l'une des revendications 1 ou 2, dans lequel l'alcool inférieur est du monopropylèneglycol.

**Office européen des brevets**

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 429 822 (P. GUALANDI)<br>* Page 2, lignes 35-38; page 3, lignes 25-33 * | 1-3 | A 61 L 9/04<br>A 61 L 9/01 |
| X | US-A-4 146 566 (C.J. GAISER)<br>* Colonne 6, lignes 28-29 * | 1,2 | |
| X | GB-A-1 570 608 (DOULTON)<br>* Revendications 1,3 * | 1,2 | |
| X | FR-A-1 602 575 (R. ARIES)<br>* Page 1, lignes 16-23 * | 1,2 | |
| X | US-A-2 778 774 (D. BUSLIK)<br>* Colonne 2, lignes 58-60 * | 1 | |

**DOMAINES TECHNIQUES RECHERCHES (Int. Cl. 3)**

A 61 L 9/04
A 61 L 9/01

Le présent rapport de recherche a été établi pour toutes les revendications

| Lieu de la recherche<br>LA HAYE | Date d'achèvement de la recherche<br>06-09-1983 | Examinateur<br>PELTRE CHR. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82